# EUROPEAN PATENT APPLICATION

(11) **EP 4 389 149 A1**
(43) Date of publication of application: **26.06.2024**
(21) Application number: 22858459.5
(22) Date of filing: 16.08.2022
(51) Int. Cl.: A61K 47/36, A61K 9/51, A61K 39/00, A61K 39/39, A61K 45/00, A61P 35/00, B82Y 5/00

(54) **CANCER THERAPEUTIC AGENT**

(30) Priority: 17.08.2021 JP 2021132504; 06.07.2022 JP 2022109018
(71) Applicant: United Immunity, Co., Ltd., Tokyo 103-0022 (JP)
(72) Inventor: HARADA, Naozumi, Tokyo 103-0022 (JP); AKIYOSHI, Kazunari, Kyoto-shi, Kyoto 606-8501 (JP); SAWADA, Shin-ichi, Kyoto-shi, Kyoto 606-8501 (JP); MURAOKA, Daisuke, Nagasaki-shi, Nagasaki 852-8521 (JP)
(74) Representative: Hoffmann Eitle
(86) International application number: PCT/JP2022/030928
(87) International publication number: WO 2023/022141

(57) **Abstract**

The present invention provides a cancer therapeutic agent for cancer tissue(s) containing CD209-positive cells. Specifically, the present invention provides a cancer therapeutic agent for cancer tissue(s) containing CD209-positive cells, the cancer therapeutic agent containing a gel particle containing a modified polysaccharide having hydrophobic groups.

## Description

### Technical Field

The present invention relates to a cancer therapeutic agent or the like.

### Background Art

The importance of immune cells, especially macrophages present in a cancer tissue (tumor-associated macrophages, abbreviated as TAM) has been considered as a factor influencing the malignant potential of cancer. Drugs that exert their cancer therapeutic effects by inhibiting or modulating TAM function have been developed. However, there is a limitation in selectivity, and no practical drugs in terms of efficacy or safety exist. Accordingly, there is demand for a technology that can selectively deliver a drug to TAMs.

It is known that CD209 (also known as DC-SIGN), a polysaccharide-binding immune receptor protein (lectin), is selectively expressed in some TAMs and correlates well with cancer stage or prognosis. CD209 is considered to be a good target of a drug delivery to TAMs.

Hydrophobic polysaccharides such as cholesteryl pullulan can self-assemble to form nanogel, have high biocompatibility, function as a protein carrier, and have an ability to inhibit decomposition and aggregation of protein. Accordingly, it has been reported that hydrophobic polysaccharides are formed into a composite with protein, and used as an antigen carrier (Non-patent Literature 1).

### Citation List

### Non-patent Literature

NPL 1: Muraoka, D. et al., ACS Nano, 8 (9), 9209-9218, 2014

### Summary of Invention

### Technical Problem

The present invention aims to provide a cancer therapeutic agent for a cancer tissue containing CD209-positive cells.

### Solution to Problem

In view of the above problems, the present inventors conducted extensive research and found that a gel particle containing a modified polysaccharide having hydrophobic groups selectively binds to and is incorporated into CD209-positive cells. Based on this finding, the present inventors conducted further research and accomplished the present invention. Specifically, the present invention comprises the following aspects.
Item 1. A cancer therapeutic agent for a cancer tissue containing CD209-positive cells, the cancer therapeutic agent containing a gel particle containing a modified polysaccharide having hydrophobic groups.
Item 2. The cancer therapeutic agent according to Item 1, wherein the gel particle is a nanogel particle.
Item 3. The cancer therapeutic agent according to Item 1 or 2, wherein a polysaccharide that forms the modified polysaccharide contains at least one member selected from the group consisting of pullulan, mannan, and dextran.
Item 4. The cancer therapeutic agent according to any one of Items 1 to 3, wherein the polysaccharide that forms the modified polysaccharide contains pullulan.
Item 5. The cancer therapeutic agent according to any one of Items 1 to 4, wherein the hydrophobic group contains a hydrophobic group having a sterol skeleton.
Item 6. The cancer therapeutic agent according to any one of Items 1 to 5, wherein the modified polysaccharide has a weight average molecular weight of 5000 to 2,000,000.
Item 7. The cancer therapeutic agent according to any one of Items 1 to 6, wherein the gel particle has a weight average particle size of 20 to 200 nm.
Item 8. The cancer therapeutic agent according to any one of Items 1 to 7, wherein the gel particle contains drug(s).
Item 9. The cancer therapeutic agent according to Item 8, wherein the drug contains at least one member selected from the group consisting of an adjuvant, a cancer antigen, an anticancer agent, and a nucleic acid medicine.
Item 10. The cancer therapeutic agent according to any one of Items 1 to 9, wherein the CD209-positive cells are a macrophage.
Item 11. The cancer therapeutic agent according to any one of Items 1 to 10, for use in administration to a patient having a cancer tissue containing CD209-positive cells, the cancer tissue being selected from a patient with cancer tissue(s).
Item 12. The cancer therapeutic agent according to any one of Items 1 to 11, for use in combination with drug(s).
Item 13. A delivery carrier to CD209-positive cells, containing a gel particle containing a modified polysaccharide having hydrophobic groups.
Item 14. The delivery carrier according to Item 13, wherein CD209-positive cells are present in a cancer tissue.
Item 15. An agent for detecting a tissue containing CD209-positive cells, containing the delivery carrier according to Item 13 or 14 and a contrast agent.
Item 15. A companion diagnostic drug for the cancer therapeutic agent according to any one of Items 1 to 12, containing CD209-binding molecules.
Item 1A. A method of treating cancer, comprising administering to a patient having cancer tissue(s) containing CD209-positive cells, a gel particle containing a modified polysaccharide having hydrophobic groups.
Item 1B. The method according to Item 1A, comprising selecting a patient with cancer tissue(s) containing CD209-positive cells from the patient with cancer tissue(s), and administering to the selected patient a gel particle containing a modified polysaccharide having hydrophobic groups.
Item 1C. A gel particle containing a modified polysaccharide having hydrophobic groups or a formulation containing the gel particle, for use as a cancer therapeutic agent for a cancer tissue containing CD209-positive cells.
Item 1D. Use of a gel particle containing a modified polysaccharide having hydrophobic groups or a formulation containing the gel particle for the production of a cancer therapeutic agent for a cancer tissue containing CD209-positive cells.
Item 1E. Use of a gel particle containing a modified polysaccharide having hydrophobic groups or a formulation containing the gel particle as a cancer therapeutic agent for a cancer tissue containing CD209-positive cells.
Item 2A. A method of delivering a drug or a contrast agent to CD209-positive cells, comprising allowing the drug or contrast agent to be mounted on a gel particle containing a modified polysaccharide having hydrophobic groups, and administering the drug or contrast agent.
Item 2B. The delivering method according to Item 2A, wherein the CD209-positive cells are present in a cancer tissue.
Item 2C. A gel particle containing a modified polysaccharides having hydrophobic groups or a formulation containing the gel particle for use as a delivery carrier to CD209-positive cells.
Item 2D. Use of a gel particle containing a modified polysaccharide having hydrophobic groups or a formulation containing the gel particle, for the production of a delivery carrier to CD209-positive cells.
Item 2E. Use of a gel particle containing a modified polysaccharide having hydrophobic groups or a formulation containing the gel particle as a delivery carrier to CD209-positive cells.
Item 3A. A method of detecting a tissue containing CD209-positive cells, the method comprising administering the delivery carrier according to Item 13 or 14 and a contrast agent or a formulation containing the delivery carrier and the contrast agent to a subject having tissue(s) containing CD209-positive cells.
Item 3B The delivery carrier according to Item 13 or 14 and a contrast agent, or a formulation containing the delivery carrier and the contrast agent, for use as an agent for detecting tissue(s) containing CD209-positive cells.
Item 3C. Use of the delivery carrier according to Item 13 or 14 and a contrast agent or a formulation comprising the delivery carrier and the contrast agent for the production of an agent for detecting tissue(s) containing CD209-positive cells.
Item 3D. Use of the delivery carrier according to Item 13 or 14 and a contrast agent or a formulation containing the delivery carrier and the contrast agent as an agent for detecting tissue(s) containing CD209-positive cells.

### Advantageous Effects of Invention

According to the present invention, a cancer therapeutic agent for cancer tissue containing CD209-positive cells can be provided.

### Brief Description of Drawings

Fig. 1 shows the result of Test Example 1 in which the rate of incorporation of rhodamine-labelled pullulan nanogel into cells was measured using flow cytometry.
Fig. 2 shows the result of Test Example 2 in which the rate of incorporation of rhodamine-labelled pullulan nanogel into cells was measured using flow cytometry.
Fig. 3 shows the result of Test Example 3 in which the incorporation (red) of rhodamine-labelled pullulan nanogel into cells was observed using fluorescence microscopy.
Fig. 4 shows the result of Test Example 4 in which the incorporation intensity (fluorescence intensity) of rhodamine-labelled pullulan nanogel into cells was measured using flow cytometry.
Fig. 5 shows the result of Test Example 5 in which the incorporation intensity (fluorescence intensity) of rhodamine-labelled pullulan nanogel into cells was measured using flow cytometry.
Fig. 6 shows the result of Test Example 6 in which the incorporation of rhodamine-labelled pullulan nanogel into cells was evaluated using fluorescence microscopy observation and flow cytometry.
Fig. 7 shows the result of Test Example 7 in which the incorporation of rhodamine-labelled pullulan nanogel into the cell population using flow cytometry.
Fig. 8 shows the result of Test Example 8 in which the incorporation of rhodamine-labelled pullulan nanogel was evaluated by fluorescence microscopy observation.
Fig. 9 shows the result of Test Example 9 in which the incorporation of rhodamine-labelled pullulan nanogel into the cell population was evaluated using flow cytometry.
Fig. 10 shows the result of Test Example 10 in which cell activity was evaluated in Cell Counting Kit-8. The vertical axis shows the relative percentage of viable cell counts and the horizontal axis shows the concentration of administration of a pullulan nanogel PE complex or PE. In the Example, 5a shows a case in which CMS5a cells are used, and 209b shows a case in which cells obtained by expressing mouse SIGNR1/CD209b in CMS5a cells are used.
Fig. 11 shows the result of measurement of the tumor size over time in Test Example 11. The vertical axis shows a tumor size (unit: mm³) and the horizontal axis shows the number of days after administration.

### Description of Embodiments

In the present specification, the terms "comprise" and "contain" include the concepts of "comprise," "contain," "essentially consist of," and "consist of."

In one embodiment, the present invention relates to a cancer therapeutic agent for a cancer tissue containing CD209-positive cells, containing a gel particle containing a modified polysaccharide having hydrophobic groups (sometimes referred to as "the cancer therapeutic agent of the present invention" in the present specification). This is explained below.

The modified polysaccharide is a compound obtained by modifying a polysaccharide, and is not particularly limited as long as it contains a hydrophobic group as a modifying group.

The polysaccharide that forms the modified polysaccharide (i.e., polysaccharide before modification) is not particularly limited as long as it is a polymer to which a sugar residue is glycosidically linked. Usable examples of sugar residues that form the polysaccharide contain residues derived from sugars, such as monosaccharides (e.g., glucose, mannose, galactose, and fucose), disaccharides, or oligosaccharides. The sugar residue may be 1,2-, 1,3-, 1,4-, or 1,6-glycosidically linked, and the linkage thereof may be either an α- or β-linkage. Further, the polysaccharide may be liner or branched. Preferable sugar residues are glucose residues. Preferable polysaccharides are, for example, naturally occurring or synthetic pullulan, mannan, dextran, amylose, amylopectin, and the like; preferably pullulan, mannan, dextran, and the like; more preferably pullulan, mannan, and the like; and particularly pullulan.

The weight average molecular weight of the polysaccharide is not particularly limited as long as the modified polysaccharide can form gel particles, but is 5,000 to 2,000,000, for example. The weight average molecular weight of the polysaccharide is preferably 10,000 to 1,000,000, more preferably 20,000 to 500,000, even more preferably 40,000 to 250,000, and still even more preferably 80,000 to 125,000.

The polysaccharide used can be a commercially available product or a product obtained according to a known production method.

The hydrophobic group is a group with hydrophobicity, and is not particularly limited as long as the modified polysaccharide can form gel particles. For example, the hydrophobic group is preferably a hydrophobic group having a sterol skeleton, a hydrocarbon group, or the like; and particularly preferably a hydrophobic group having a sterol skeleton.

The sterol skeleton is an alcohol in which a hydroxy group is attached to a cyclopentahydrophenanthrene ring shown in formula I. Symbols A to D in formula I represent each ring that forms the cyclopentahydrophenanthrene ring.

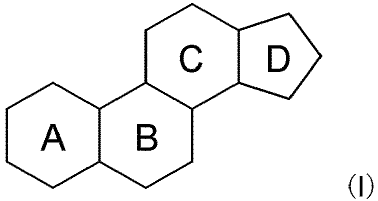

In the sterol skeleton, the cyclopentahydrophenanthrene ring may have a double bond, and the bonding position of the hydroxyl group is not limited. Preferable are either sterols with a hydroxy group at the C-3 position and a double bond in the B ring, or stanols with a hydroxy group at the C-3 position and a saturated ring. Examples of the hydrophobic group having a sterol skeleton include groups derived from compounds with a modified sterol skeleton, for example, the ring-forming carbon is replaced by a hydrocarbon group (e.g., a C₁₋₂₀ linear or branched alkyl group). The phrase "group derived from" refers to a group of a compound from which a hydrogen atom or a functional group, such as a hydroxyl group, is removed.

Examples of the hydrophobic group having a sterol skeleton include cholesterol-derived groups, cholestanol-derived groups, lanosterol-derived groups, ergosterol-derived groups, β-sitosterol-derived groups, campesterol-derived groups, stigmasterol-derived groups, brassicasterol-derived groups, and the like. Preferred among these are sterol-derived groups, such as cholesterol-derived groups, cholestanol-derived groups, lanosterol-derived groups, and ergosterol-derived groups; and more preferred are cholesterol-derived groups.

The hydrocarbon group as the hydrophobic group is not particularly limited, and examples include C₈₋₅₀ (preferably C₁₀₋₃₀, more preferably C₁₂₋₂₀) chain (preferably linear) hydrocarbon groups (preferably alkyl groups).

The weight average molecular weight of the modified polysaccharide is not particularly limited as long as the modified polysaccharide can form gel particles, but is 5,000 to 2,000,000, for example. The weight average molecular weight of the modified polysaccharide is preferably 10,000 to 1,000,000, more preferably 20,000 to 500,000, even more preferably 40,000 to 250,000, and still even more preferably 80,000 to 125,000.

The number of hydrophobic groups contained in the modified polysaccharide is not particularly limited as long as the modified polysaccharide can form gel particles, but is, for example, 1 to 10, and preferably 1 to 5, per 100 sugar residues that form the polysaccharide.

The hydrophobic group can be linked to the polysaccharide directly or indirectly (e.g., through a linker).

The modified polysaccharide is preferably, for example, one containing 1 to 10 (preferably 1 to 5) sugar units per 100 sugar residues that form the polysaccharide, whose primary hydroxyl group is represented by formula II: -O-(CH₂)ₘCONH(CH₂)ₙNH-CO-O-R (II), wherein R is a hydrophobic group having a sterol skeleton or a hydrocarbon group, m is 0 or 1, and n is any positive integer. n is preferably 1 to 8.

The modified polysaccharide can be synthesized by or according to a known method (e.g., WO00/12564). For example, the following method can be used. First, C₁₂₋₅₀ hydroxyl group-containing hydrocarbon or sterol, and a diisocyanate compound represented by formula OCN-R¹NCO, wherein R¹ is C₁₋₅₀ hydrocarbon group, are reacted to produce an isocyanato group-containing hydrophobic compound in which one molecule of C₁₂₋₅₀ hydroxyl group-containing hydrocarbon or sterol is reacted. Then, the resulting isocyanato group-containing hydrophobic compound and a polysaccharide are further reacted to produce a hydrophobic group-containing polysaccharide containing a C₁₂₋₅₀ hydrocarbon group or a stearyl group as a hydrophobic group. The obtained reaction product is purified with a ketone solvent, whereby a high-purity hydrophobic group-containing polysaccharide can be produced.

The modified polysaccharides can be used singly or in combination of two or more.

The gel particle contains a modified polysaccharide. The term "gel particle" refers to a polymer gel particle with a hydrogel structure. The hydrogel refers to a three-dimensional network structure formed by crosslinking of hydrophilic polymers and swelling with water. In the gel particles, the modified polysaccharide is self-assembled through physical crosslinks formed based on hydrophobic interactions with the hydrophobic group, forming a three-dimensional network structure.

The shape of the gel particles is not particularly limited, but is generally spherical.

The gel particles are preferably nanosized (i.e., nanogel particles). The weight average particle size of the nanogel particles is, for example, 200 nm or less, preferably 10 to 200 nm, more preferably 15 to 200 nm, and even more preferably 20 to 200 nm. The upper limit of the weight average particle size is preferably 100 nm, more preferably 70 nm, and even more preferably 50 nm. The particle size can be measured by a dynamic light scattering method.

The gel particle may contain a substance other than the modified polysaccharide. Examples of other substances include proteins, peptides, nucleic acids, sugars, low-molecular-weight compounds, high-molecular-weight compounds, and inorganic materials, as well as complexes thereof. Specific examples of other substances include an adjuvant, a cancer antigen, an anti-cancer agent, a drug such as a nucleic acid medicine, a contrast agent, etc.

The adjuvant can be selected from inactivated bacteria or extracts of bacteria, nucleic acid lipopolysaccharides, lipopeptides, synthetic low-molecular-weight compounds, and the like; and preferably an imidazoquinoline (e.g., R848 and imiquimod), saponin (e.g., QuilA and QS21), STING agonist (e.g., cyclic di-GMP), monophosphoryl lipid, and lipopeptide are used. In addition to the above, other examples of the adjuvant include taxane-based drugs, anthracycline-based drugs, inhibitors of JAK/STAT, inhibitors of indole dioxygenase (IDO), and inhibitors of tryptophan dioxygenase (TDO). These inhibitors include compounds that have an antagonistic effect on the factor as well as neutralizing antibodies of the factor.

Examples of the cancer antigen include an antigen polypeptide, preferably an antigen polypeptide. The antigen polypeptide is an antigen or the partial peptide of the antigen that is highly expressed in a cancer cell. In some cases, the polypeptide is expressed only in a cancer cell. The antigen polypeptide can be expressed in a cancer cell or on the surface of the cancer cell.

The antigen polypeptide is not limited, and can be selected from ERK1, ERK2, MART-1/Melan-A, gp100, adenosine deaminase binding protein (ADAbp), FAP, cyclophilin b, colorectal-associated antigen (CRC)-C017-1A/GA733, carcinoembryonic antigen (CEA), CAP-1, CAP-2, etv6, AML1, prostate-specific antigen (PSA), PSA-1, PSA-2, PSA-3, prostate-specific membrane antigen (PSMA), T cell receptor/CD3-zeta chain, CD20, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5, GAGE-1, GAGE-2, GAGE-3, GAGE-4, GAGE-5, GAGE-6, GAGE-7, GAGE-8, and GAGE-9, BAGE, RAGE, LAGE-1, NAG, GnT-V, MUM-1, CDK4, tyrosinase, P53, MUC family, HER2/neu, p21ras, RCAS1, α-fetoprotein, E-cadherin, α-catenin, β-catenin, γ-catenin, p120ctn, gp100Pmel117, FRAME, NY-ESO-1, cdc27, adenomatous polyposis coli (APC) protein, fodrin, connexin 37, Ig idiotype, p15, gp75, GM2 ganglioside, GD2 ganglioside, human papillomavirus protein, smad family of tumor antigens, lmp-1, P1A, EBNA (EBV nuclear antigen)-1, brain glycogen phosphorylase, SSX-1, SSX-2 (HOM-MEL-40), SSX-1, SSX-4, SSX-5, SCP-1, CT-7, CD20, c-erbB-2, and partial peptides thereof.

Examples of anticancer agents include alkylating agents, antimetabolites, microtubule inhibitors, antibiotic anticancer agents, topoisomerase inhibitors, platinum preparations, molecular-targeted drugs, hormonal agents, biologics, and the like.

Examples of alkylating agents include cyclophosphamide, ifosfamide, nitrosourea, dacarbazine, temozolomide, nimustine, busulfan, melphalan, procarbazine, ranimustine, and the like.

Examples of antimetabolites include enocitabine, carmofur, capecitabine, tegafur, tegafur uracil, tegafur gimeracil oteracil potassium, gemcitabine, cytarabine, cytarabine ocfosfate, nelarabine, fluorouracil, fludarabine, pemetrexed, pentostatin, methotrexate, cladribine, doxifluridine, hydroxycarbamide, mercaptopurine, and the like.

Examples of microtubule inhibitors include alkaloid anticancer agents, such as vincristine; and taxane anticancer agents, such as docetaxel and paclitaxel.

Examples of antibiotic anticancer agents include mitomycin C, doxorubicin, epirubicin, daunorubicin, bleomycin, actinomycin D, aclarubicin, idarubicin, pirarubicin, peplomycin, mitoxantrone, amrubicin, zinostatin stimalamer, and the like.

Examples of topoisomerase inhibitors include CPT-11, irinotecan, and nogitecan, all of which have topoisomerase I inhibitory action; and etoposide and sobuzoxane, both of which have topoisomerase II inhibitory action.

Examples of platinum preparations include cisplatin, nedaplatin, oxaliplatin, carboplatin, and the like.

Examples of hormonal agents include dexamethasone, finasteride, tamoxifen, astrozole, exemestane, ethinyl estradiol, chlormadinone, goserelin, bicalutamide, flutamide, prednisolone, leuprorelin, letrozole, estramustine, toremifene, fosfestrol, mitotane, methyltestosterone, medroxyprogesterone, mepitiostane, and the like.

Examples of biologics include interferon-a, -β, and -γ, interleukin-2, ubenimex, dried BCG, an extracellular toxin, and the like. Examples of the extracellular toxin include an extracellular toxin (protein) producing bacteria, which is Pseudomonas Exotoxin (PE). The PE has an enzyme activity of NAD+-diphthamide-ADP-ribosyl transferase, and inhibits protein synthesis and exhibits high toxicity. So far, those in which PE is conjugated to an antibody, such as a CD22 antibody, have been approved as an anticancer drug.

Examples of molecular-targeted drugs include rituximab, alemtuzumab, trastuzumab, cetuximab, panitumumab, imatinib, dasatinib, nilotinib, gefitinib, erlotinib, temsirolimus, bevacizumab, VEGF trap, sunitinib, sorafenib, tosituzumab, bortezomib, gemtuzumab ozogamicin, ibritumomab ozogamicin, ibritumomab tiuxetan, tamibarotene, tretinoin, and the like. In addition to the molecular-targeted drugs specified herein, other examples of molecular-targeted drugs include inhibitors targeting angiogenesis, such as human epidermal growth factor receptor 2 inhibitors, epidermal growth factor receptor inhibitors, Bcr-Abl tyrosine kinase inhibitors, epidermal growth factor tyrosine kinase inhibitors, mTOR inhibitors, and vascular endothelial growth factor receptor 2 inhibitors (α-VEGFR-2 antibody); various tyrosine kinase inhibitors, such as MAP kinase inhibitors; cytokine-targeted inhibitors, proteasome inhibitors, and antibody-anticancer agent formulations. These inhibitors also include antibodies.

Nucleic acid medicines are active pharmaceutical ingredients that are nucleic acids and are not particularly limited as long as the nucleic acid medicines are active pharmaceutical ingredients.

The contrast agent is not particularly limited as long as it is a substance capable of visualizing the location. Examples of the contrast agent include X-ray contrast agents, such as barium sulfate, bismuth bicarbonate, bismuth oxide, zirconium oxide, ytterbium fluoride, iodoform, barium apatite, barium titanate, lanthanum glass, barium glass, strontium glass, etc.; computed tomography (CT) contrast agents, such as iodine contrast agents; MRI contrast agents such as gadolinium formulations and super paramagnetic iron oxide (SPIO) formulations; radioactive isotopes for single photon emission computed tomography (SPECT) such as technetium 99m (99mTc) and molybdenum 99 (99Mo); and various other radioactive isotopes or materials containing the isotopes.

The content of other substances is, for example, 0 to 10000 parts by mass, 0 to 1000 parts by mass, 0 to 500 parts by mass, 0 to 100 parts by mass, 0 to 50 parts by mass, or 0 to 10 parts by mass, based on 100 parts by mass of the content of the modified polysaccharide.

The gel particle can be produced according to a known method. The gel particle can be produced by subjecting a modified polysaccharide-containing solution to sonication, or by adding a denaturant, such as urea, DMSO, or a surfactant, to the solution and then removing the denaturant by dialysis. In terms of ease, the former method is more preferable. The latter method can be performed by or according to a known method.

The modified polysaccharide-containing solution can be prepared by dissolving the modified polysaccharide in a solvent. As the solvent, for example, water or organic solvents such as DMSO can be used. The modified polysaccharide solution can be generally prepared by using water as the solvent. In this case, it is preferable to use a buffer solution, such as PBS, in place of water.

The concentration of the modified polysaccharide in the modified polysaccharide-containing solution is not particularly limited, but is, for example, 5 to 100 uM, preferably 5 to 80 uM, and more preferably 8 to 50 uM, from the viewpoint of the formation efficiency of gel particles.

Sonication can be performed, for example, by applying ultrasonic waves to a plastic tube containing the modified polysaccharide-containing solution and fixed on water in an ultrasonic bath tank. The sonication conditions are not particularly limited, but are, for example, 10 to 40°C (preferably 20 to 35°C), 10 to 50 kHz (preferably 20 to 40 kHz), 30 to 200 W (preferably 70 to 150 W), and 2 to 30 minutes (preferably 5 to 15 minutes).

In an embodiment, the gel particle is used as a cancer therapeutic agent for cancer tissue containing CD209-positive cells. The gel particle binds to a cell expressing CD209 as a target and is incorporated. This allows the component contained in the gel particle to be selectively transported to cancer tissue containing CD209-positive cells. Specifically, for example, a drug (an adjuvant, cancer antigen, anti-cancer agent, nucleic acid drug, etc.) contained in the gel particle can be selectively transferred to cancer tissue containing CD209-positive cells to exert immunostimulatory and anticancer effects, thus treating cancer. Specifically, for example, a cancer antigen contained in a gel particle is incorporated in CD209-positive cells (preferably macrophages and tumor-associated macrophages), allowing an antigen presentation to the cell to activate a lymphocyte such as T cells to treat cancer.

Since the gel particle is bound to cells expressing CD209 as a target on cell membrane, and is incorporated, it is possible to use the gel particle as a delivery carrier (delivery carrier of the present invention) to CD209-positive cells. The CD209-positive cells are preferably present in cancer tissue. By administering the delivery carrier of the present invention together with a substance (other substance that the gel particle can contain (for example, an adjuvant, a cancer antigen, an anti-cancer agent, a drug such as a nucleic acid medicine, a contrast agent, etc.) that delivers the delivery carrier of the present invention (preferably by allowing other substances to be mounted (e.g., bound or held) onto the gel particle), the other substances can be delivered to the CD209-positive cells (preferably cancer tissue containing CD209-positive cells). In one embodiment, the delivery carrier is used, for example, as a drug delivery carrier.

When the delivery carrier of the present invention is used together with a contrast agent (preferably with the contrast agent mounted on (e.g., bound to or held on) a gel particle), it can be used as an agent for detecting a tissue containing CD209-positive cells (preferably cancer tissue containing CD209-positive cells). Accordingly, in one embodiment, the present invention relates to an agent for detecting tissue(s) containing CD209-positive cells, containing the delivery carrier of the present invention and the contrast agent. The detecting agent can be used as a detecting agent used for MRI, PET, and CT depending on the type of the contrast agent. The detecting agent can be used to visualize tissue(s) containing CD209-positive cells.

CD209 is a C-type lectin receptor, which is also referred to as DC-SIGN in a human and is expressed on the cell surface. CD209 in other animals can be determined based on sequence identity with human CD209/DC-SIGN. For example, the functional homologue of CD209 in mice is CD209b, which is also referred to as SIGNR1.

The cancer tissue containing CD209-positive cells can be determined, for example, by selecting a specific cell group (preferably macrophages) in the cancer tissue and determining the amount of CD209-positive cells contained in the cancer tissue. The selection and determination can be performed according to a known method. Examples include an immunohistochemical staining method. In this method, CD209-positive cells in cancer tissue are stained with an anti-human CD209 antibody (fluorescence or chromogenic method), and whether the cell is positive or negative is determined based on the amount of dye (e.g., fluorescence amount or visible light amount) emitted by the stained cell. Such staining can typically be performed using a CD209-binding molecule. Accordingly, in one embodiment, the present invention relates to a companion diagnostic drug (the diagnostic drug of the present invention) for the cancer therapeutic agent of the present invention, containing a CD209-binding molecule. By a method including the step of bringing a CD209-binding molecule into contact with a cell in a cancer tissue of a subject, efficacy against the cancer therapeutic agent of the present invention can be predicted.

The CD209-binding molecule is not particularly limited as long as it is a molecule bindable (preferably specifically bindable) to CD209. Examples of the CD209-binding molecule include an antibody, more specifically, immunoglobulin, Fab, F(ab')2, minibody, scFv-Fc, Fv, scFv, diabody, triabody, tetrabody, monobody, etc.

The series of operations of selection and determination can be performed using microscopic observation. The determination as to whether the cell is positive or negative is based on the amount of dye emitted by the stained cell, according to a known method. This determination can be made according to a known technique. For example, the amount of dye emitted by a negative control cell (specifically, for example, a cell stained with an isotype control antibody) is used as the amount of dye in the background. Based on the amount of dye sufficiently higher than that in the background, a cell with a lower amount of dye than the standard is determined to be a negative cell, and a cell with higher fluorescence than the standard is determined to be a positive cell. As a simpler method, reverse transcription-quantitative PCR for CD209 on mRNA extracted from a cancer tissue is performed to conduct determination based on the amount of CD209 mRNA.

It is preferable to select a patient with cancer tissue(s) containing CD209-positive cells from patients with cancer tissue(s), and to administer the cancer therapeutic agent or the delivery carrier of the present invention to the selected patient. Accordingly, it is preferable to use the cancer therapeutic agent or the delivery carrier of the present invention to be administered to a patient with cancer tissue(s) containing CD209-positive cells, who is selected from patients with cancer tissue(s).

The cancer therapeutic agent or delivery carrier of the present invention is not limited as long as it contains a gel particle. The cancer therapeutic agent or delivery carrier of the present invention may further contain other components, if necessary. The other components are not particularly limited as long as they are pharmaceutically acceptable components. Examples of other components include additives in addition to components with pharmacological effects. Examples of additives include bases, carriers, solvents, dispersants, emulsifiers, buffers, stabilizers, excipients, binders, disintegrators, lubricants, thickeners, moisturizers, coloring agents, flavoring agents, chelating agents, and the like.

In addition to the gel particle, the cancer therapeutic agent or the delivery carrier of the present invention can contain other substances (e.g., an adjuvant, a cancer antigen, an anti-cancer agent, a drug such as a nucleic acid medicine, a contrast agent, etc.) that the gel particle can contain. The cancer therapeutic agent or the delivery carrier of the present invention can also be used in combination with other substances (e.g. an adjuvant, a cancer antigen, an anti-cancer agent, a drug such as a nucleic acid medicine, a contrast agent, etc.) that can be contained in the gel particle.

The application object of the cancer therapeutic agent or delivery carrier of the present invention is not particularly limited. Examples of mammals include humans, monkeys, mice, rats, dogs, cats, rabbits, pigs, horses, cows, sheep, goats, deer, and the like. Further, examples of cells include animal cells and the like. The type of cell is also not particularly limited, and examples include blood cells, hematopoietic stem cells/progenitor cells, gametes (sperm and egg), fibroblasts, epithelial cells, vascular endothelial cells, neurons, hepatocytes, keratinocytes, muscle cells, epidermal cells, endocrine cells, ES cells, iPS cells, tissue stem cells, cancer cells, and the like.

The target cancer of the cancer therapeutic agent or delivery carrier of the present invention is not particularly limited. Examples include leukemia (including chronic lymphocytic leukemia and acute lymphocytic leukemia), lymphoma (including non-Hodgkin's lymphoma, Hodgkin's lymphoma, T-cell lymphoma, B-cell lymphoma, Burkitt's lymphoma, malignant lymphoma, diffuse lymphoma, and follicular lymphoma), myeloma (including multiple myeloma), breast cancer, colon cancer, kidney cancer, gastric cancer, ovarian cancer, pancreatic cancer, cervical cancer, endometrial cancer, esophageal cancer, liver cancer, head and neck squamous epithelial cancer, skin cancer, malignant melanoma, urinary tract cancer, prostate cancer, villous cancer, pharyngeal cancer, laryngeal cancer, thecoma, male germinoma, endometrial hyperplasia, endometriosis, embryoma, fibrosarcoma, Kaposi sarcoma, hemangioma, cavernous hemangioma, hemangioblastoma, retinoblastoma, astrocytoma, neurofibroma, oligodendroglioma, medulloblastoma, neuroblastoma, glioma, rhabdomyosarcoma, medulloblastoma, osteogenic sarcoma, leiomyosarcoma, thyroid sarcoma, Wilms tumor, and the like.

The cancer therapeutic agent or delivery carrier of the present invention can have any dosage form, and examples include oral dosage forms, such as tablets (including orally disintegrating tablets, chewable tablets, foaming tablets, troches, jelly drops, etc.), pills, granules, subtle granules, powders, hard capsules, soft capsules, dry syrups, liquids (including drinkable preparations, suspensions, and syrups), and jellies; and parenteral dosage forms, such as injectable preparations (e.g., drip injections (intravenous drip injections etc.), intravenous injections, intramuscular injections, subcutaneous injections, and intradermal injections), external preparations (e.g., ointments, poultices, and lotions), suppositories, inhalants, eye drops, eye ointments, nasal drops, ear drops, and liposome preparations.

The route of administration of the cancer therapeutic agent or delivery carrier of the present invention is not particularly limited as long as desired effects are obtained. Examples include enteral administration, such as oral administration, tube feeding, and enema administration; parenteral administration, such as intravenous administration, intraarterial administration, intramuscular administration, intracardiac administration, subcutaneous administration, intradermal administration, and intraperitoneal administration; and the like.

The content of the active ingredient in the cancer therapeutic agent or the delivery carrier of the present invention varies depending on the usage mode, application object, the state of the applied object, etc., and is not limited. For example, the content of the active ingredient is 0.0001 to 100 wt%, and preferably 0.001 to 50 wt%.

The dose of the cancer therapeutic agent or delivery carrier of the present invention when administered to an animal is not particularly limited as long as it is an effective amount for expressing drug effects. In the case of oral administration, the weight of the active ingredient is generally 0.1 to 1000 mg/kg body weight per day, and preferably 0.5 to 500 mg/kg body weight per day. In the case of parenteral administration, the weight of the active ingredient is 0.01 to 100 mg/kg body weight per day, and preferably 0.05 to 50 mg/kg body weight per day. The above dose can be suitably increased or decreased according to the age, pathological conditions, symptoms, etc.

### Examples

The present invention is described in detail below based on Examples; however, the present invention is not limited to these Examples.

### Preparation Example 1: Preparation of pullulan nanogel

According to a previously reported document (Macromolecules 1993, 23, 3062-3068), cholesterol-modified pullulan (CHP) was produced by introducing 1.2 cholesterols per 100 monosaccharides to pullulan having a weight average molecular weight of 100,000.

CHP was added to PBS to a concentration of 1 mg/mL, and dissolved by stirring with a stirrer at 25°C for 15 hours. Then, ultrasonic waves were intermittently applied for 6 minutes using a probe-type ultrasonic wave-generating device. After the solution was centrifuged at 25°C and 20000 g for 30 minutes, the supernatant was sterilized by filtration through a 0.22-um filter to obtain pullulan nanogel (nanogel weight average molecular weight: 450,000).

The average particle size of the obtained pullulan nanogel was measured by a cumulant method analysis using a Dynamic Light Scattering (DLS). The results indicate that the pullulan nanogel has an average particle size of 32.2 ± 2.1 nm and a polydispersity index of 0.26 ± 0.01.

### Test Example 1. Binding of pullulan nanogel to mouse CD209

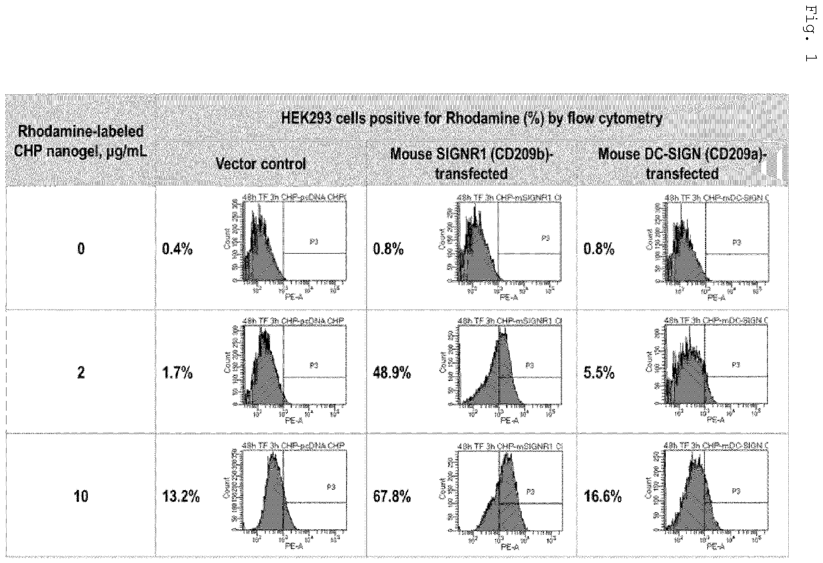

An expression vector encoding the cDNA of mouse SIGNR1/CD209b or mouse DC-SIGN/CD209a was transiently introduced into HEK293 cells by lipofection. The same procedure was performed using an expression vector free of cDNA as a control (vector control). After 48 hours, rhodamine-labelled pullulan nanogel was administered to cells at a concentration shown in Fig. 1, and subjected to incubation at 37°C for 3 hours. The cell was washed with a cold PBS, and isolated with an EDTA-containing cold PBS, followed by suspension to obtain single cells. The rate of incorporation of the rhodamine-labelled pullulan nanogel into cells was measured using flow cytometry.

Fig. 1 shows the results. In the mouse SIGNR1/CD209b-expressing cells, the rhodamine-labeled pullulan nanogel was incorporated into 48.9% (in the case of 2 µg pullulan nanogel/mL) or 67.8% (in the case of 10 µg pullulan nanogel/mL) of the cells while in the mouse DC-SIGN/CD209a expressing cells, the rhodamine-labeled pullulan nanogel was incorporated into the cells at a low value such as 5.5% (in the case of 2 µg pullulan nanogel/mL) or 16.6% (in the case of 10 µg pullulan nanogel/mL) of the cells. Mouse SIGNR1/CD209b is functionally close to human DC-SIGN/CD209. In this case, the introduction efficiency evaluated in the transient expression of the expression vector encoding the cDNA of Green Fluorescent Protein (GFP) was about 40%.

### Test Example 2. Binding of pullulan nanogel to human CD209

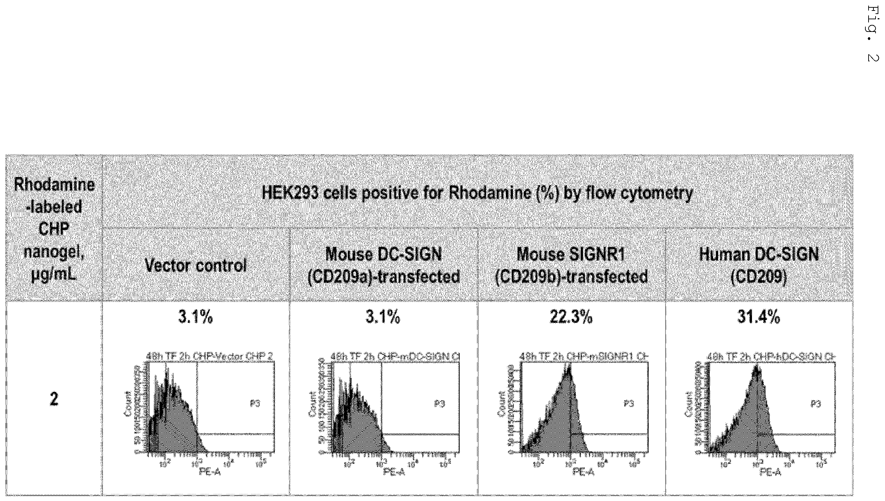

In the same manner as in Test Example 1, an expression vector encoding cDNA of mouse SIGNR1/CD209b, mouse DC-SIGN/CD209a, or human DC-SIGN/CD209 was transiently introduced into HEK293 cells by lipofection. The same procedure was performed using an expression vector free of cDNA as a control (vector control). After 48 hours, the rhodamine-labelled pullulan nanogel was administered to cells at a concentration shown in Fig. 2, followed by incubation at 37°C for 2 hours. Cells were washed with a cold PBS, and isolated with an EDTA-containing cold PBS, followed by suspension to obtain single cells. The rate of incorporation of rhodamine-labelled pullulan nanogel into cells was measured using flow cytometry.

The results are shown in Fig. 2. The rhodamine-labeled pullulan nanogel was incorporated into 3.1% of mouse DC-SIGN/CD209a-expressing cells, 22.3% of mouse SIGNR1/CD209b-expressing cells, and 31.4% of human DC-SIGN/CD209-expressing cells. In this case, the introduction efficiency evaluated in the transient expression of the expression vector encoding the cDNA of Green Fluorescent Protein (GFP) was about 29%.

### Test Example 3. Specific binding of pullulan nanogel to CD209

An expression vector encoding cDNA of C-type lectin was introduced into COS7 cells by lipofection. The same procedure was performed using an expression vector free of cDNA as a control (vector control). After 48 hours, rhodamine-labelled pullulan nanogel was administered at a concentration of 2 ug/mL to cells, followed by incubation at 37°C for 2 hours. The cells were washed with cold PBS, and isolated with TrypLE (Thermo Scientific), followed by suspension to obtain single cells. Fluorescence microscopy was used to observe the incorporation (red) of rhodamine-labelled pullulan nanogel into cells.

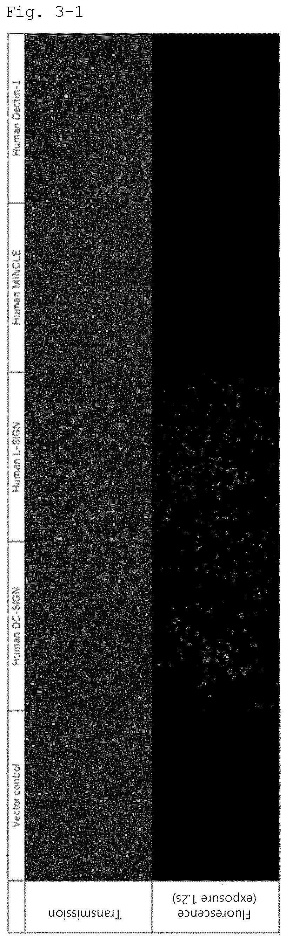

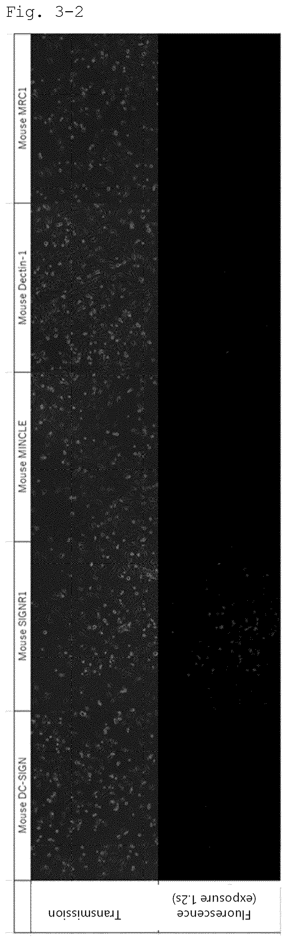

Fig. 3-1 shows the results of human C-type lectin-expressing cells and Fig. 3-2 shows the results of mouse C-type lectin-expressing cells. The incorporation of rhodamine-labelled pullulan nanogel was observed only in cells expressing human DC-SIGN/CD209, human L-SIGN, and mouse SIGNR1/CD209b, and was not observed in cells expressing C-type lectin.

### Test Example 4. Binding of polysaccharide nanogel and linear polysaccharide to CD209

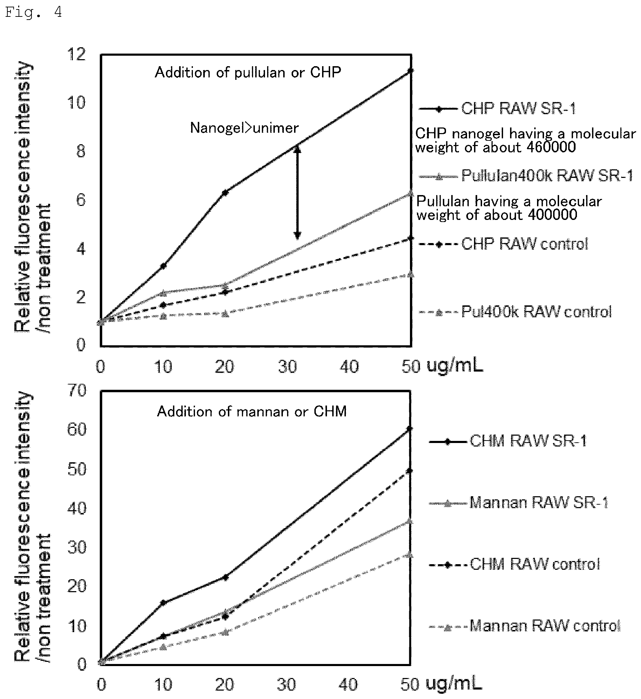

Rhodamine-labelled pullulan nanogel (CHP in Fig. 4) or rhodamine-labelled pullulan (linear, unimer) (Pullulan 400K in Fig. 4) was administered at a concentration shown in Fig. 4 to mouse RAW 264.7 cells stably expressing a mouse SIGNR1/CD209b gene (RAW SR-1 in Fig. 4), followed by incubation at 37°C for 3 hours. The cells were isolated with a scraper and suspended to form single cells. The incorporation intensity (fluorescence intensity) of rhodamine-labelled pullulan nanogel into cells was measured using flow cytometry. A test was performed in the same manner using mannan (linear, unimer) and mannan nanogel.

The results are shown in Fig. 4. Both of pullulan nanogel and pullulan were incorporated into RAW SR-1 cells in a concentration-dependent manner. The incorporation of pullulan nanogel (nanoparticle form) was higher than that of pullulan (unimer, linear).

### Test Example 5. SIGNR1 dependency of binding of pullulan nanogel to RAW SR-1 cells

The same experiment as in Test Example 4 was performed. However, cells were treated with a SIGNR1-specific antibody (clone 22D1 (IgG) or clone ERTR9 (IgM)) for 1 hour before the addition of rhodamine-labelled pullulan nanogel.

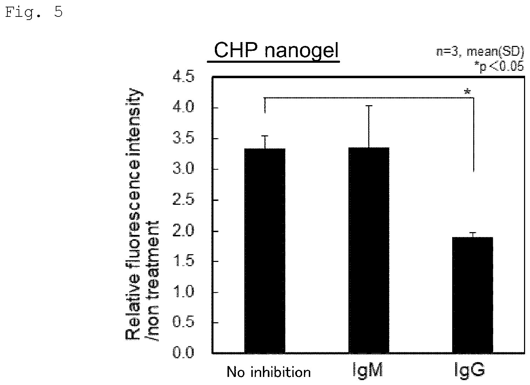

The results are shown in Fig. 5. In the cells treated with a 22D1 antibody (IgG), a significant reduction in the incorporation of pullulan nanogel was observed. It was considered that since 22D1 binds to the glycan recognition site of SIGNR1/CD209b, 22D1 interferes with the binding of SIGNR1/CD209b to the pullulan nanogel, thus inhibiting the incorporation of pullulan nanogel. It was considered that since ERTR9 does not bind to the glycan recognition site of SIGNR1/CD209b, the incorporation of pullulan nanogel was not inhibited. The results indicated that the pullulan nanogel and SIGNR1 were specifically bound.

### Test Example 6. Specific binding of pullulan nanogel to CD209

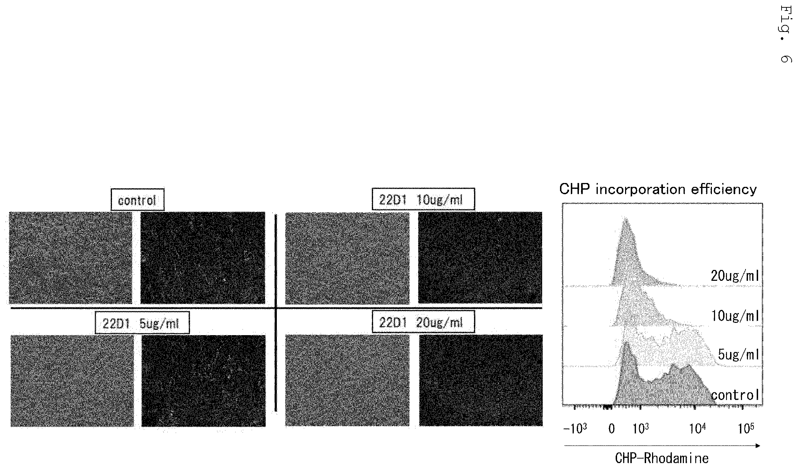

An expression vector encoding the cDNA of mouse SIGNR1/CD209b was transiently introduced into Mouse fibrosarcoma cell line CMS5a cells by electroporation. The following day, rhodamine-labelled pullulan nanogel was administered at a concentration of 2 ug/mL or an anti-SIGNR1 antibody (22D1) was administered at a concentration shown in Fig. 6 to the cells, followed by incubation at 37°C for 2 hours. The cells were isolated with trypsin and suspended to form single cells. The incorporation of rhodamine-labelled pullulan nanogel into cells was evaluated using fluorescence microscopy observation and flow cytometry.

The results are shown in Fig. 6. The incorporation of rhodamine-labelled pullulan nanogel was clearly observed in SIGNR1/CD209b-expressing cells (control in Fig. 6). In contrast, the addition of a 22D1 antibody attenuated the incorporation of rhodamine-labelled pullulan nanogel in a concentration-dependent manner. As in Fig. 5, it was supported that the pullulan nanogel was specifically bound to SIGNR1/CD209b.

### Test Example 7. Selective incorporation of pullulan nanogel into tumor-associated macrophage (TAM)

Mouse colorectal cancer cell line CT 26 cells were subcutaneously transfected with normal BALB/c mice (7-week-old, female), and 8 days later, the tumor was collected and crushed with GentleMACS (Miltenyi Biotec) to obtain a cell suspension, which was then cultured in RPMI 1640 medium containing 10% fetal bovine serum on a culture plate. Rhodamine-labeled pullulan nanogel was administered thereto at a concentration of 2 µg/mL, followed by incubation for 2 hours at 37°C. Thereafter, the cells were isolated with trypsin and suspended to form single cells. The cells were stained with an anti-CD45 antibody, CD11b antibody, Gr1 antibody, SIGNR1/CD209b antibody, and MHC class II antibody to evaluate the incorporation of rhodamine-labeled pullulan nanogel into the cell population using flow cytometry.

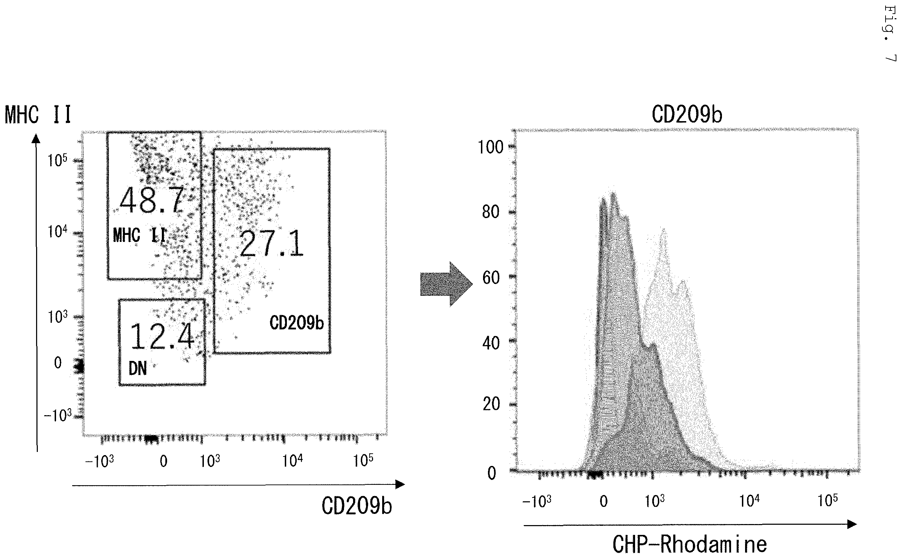

The results are shown in Fig. 7. Among myeloid cells, incorporation of rhodamine-labeled pullulan nanogel at a high level was clearly observed in CD11b+Gr1-CD209b+cells (CD209b-positive TAM) (Fig. 7 on the right).

### Test Example 8. Selective incorporation of pullulan nanogel into tumor-associated macrophage (TAM)

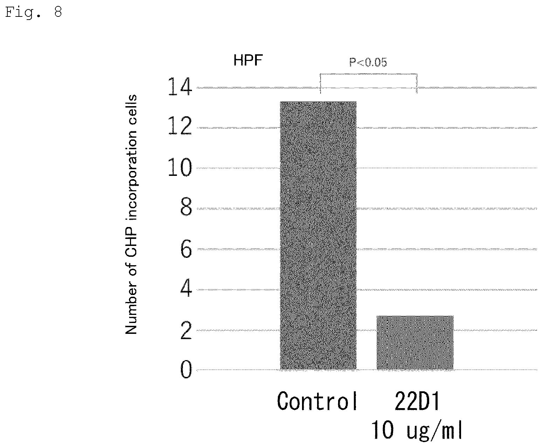

Mouse colorectal cancer cell line CT 26 cells were subcutaneously transfected with normal BALB/c mice (7-week-old, female), and 8 days later, the tumor was collected and crushed with GentleMACS (Miltenyi Biotec) to obtain a cell suspension, which was then cultured in RPMI 1640 medium containing 10% fetal bovine serum on a culture plate. Rhodamine-labeled pullulan nanogel was administered thereto at a concentration of 2 µg/mL, followed by incubation for 2 hours at 37°C. During administration, a SIGNR1 antibody (22D1) was simultaneously added at a concentration shown in Fig. 8. The observation using a fluorescence microscope was conducted to evaluate the incorporation of rhodamine-labeled pullulan nanogel. The number of intensely luminescent cells in a field of x400 was taken as the number of incorporation cells.

The results are shown in Fig. 8. In the presence of the 22D1 antibody, the incorporation of rhodamine-labelled pullulan nanogel was significantly inhibited.

### Test Example 9. Selective incorporation of pullulan nanogel into tumor-associated macrophage (TAM)

The mouse colon cancer cell line CT26 cells were transfected subcutaneously with normal BALB/c mice (7-week-old, female), and 8 days later, rhodamine-labelled pullulan nanogel (600 µg) was intravenously administered. During administration, a SIGNR1 antibody (22D1) was administered simultaneously at the concentration. Six hours later, the tumor was collected, and crushed with GentleMACS (Miltenyi Biotec) to obtain a cell suspension. Thereafter, the suspension was stained with an anti-CD45 antibody, a CD11b antibody, a Gr1 antibody, a CD209b antibody, a CD206 antibody, and an MHC class II antibody, and flow cytometry was used to evaluate the incorporation of the rhodamine-labelled pullulan nanogel into the cell population.

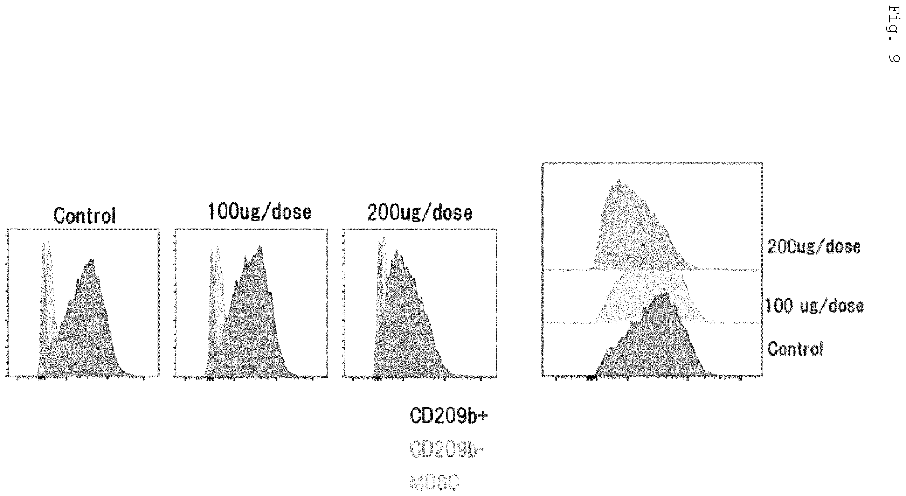

The results are shown in Fig. 9. High incorporation of rhodamine-labelled pullulan nanogel was clearly observed in CD11b+Gr1-CD209b+cells (CD209-positive TAM) (Fig. 9 on the left). In contrast, the SIGNR1 antibody inhibited the incorporation of pullulan nanogel into the same cells in a concentration-dependent manner.

### Test Example 10. Cytotoxicity by pullulan nanogel Pseudomonas Exotoxin (PE) complex

An expression vector encoding the cDNA of mouse SIGNR1/CD209b was transiently introduced into Mouse fibrosarcoma cell line CMS5a cells by electroporation. The following day, a pullulan nanogel PE complex or PE was administered at a concentration of 0.3, 1, 3, 9, 10, 30, or 90 pg/mL, followed by incubation at 37°C for 1 hour. 1 hour later, the cells were washed with 10% FCS RPMI and then cultured in 10%FCS RPMI for 24 hours, after which the cell activity was evaluated with cell counting Kit-8.

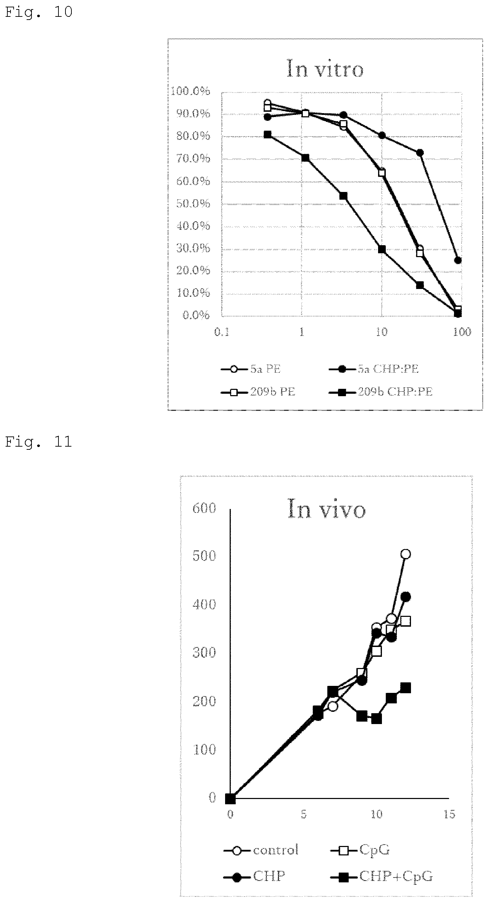

The results are shown in Fig. 10. When PE alone was added, concentration-dependent cytotoxic activity was observed regardless of the expression of SIGNR1/CD209b, whereas the pullulan nanogel PE complex showed predominantly cytotoxic activity in SIGNR1/CD209b-expressing cells.

### Test Example 11. Cytotoxicity by pullulan nanogel PE complex

The mouse colon cancer cell line CMS5a cells were subcutaneously transplanted into normal BALB/c mice (8-week-old, female), and 8, 10, and 12 days later, the pullulan nanogel PE complex and PE (0.1 pg) were administered intravenously together with CpG (50 ug). The tumor size was measured over time.

The results are shown in Fig. 11. The combination use of the pullulan nanogel PE complex and CpG attained a predominant tumor growth inhibitory effect as compared to a CpG alone administration group and a pullulan nanogel PE alone administration group. The PE administration mice were dead within 24 hours after administration.

## Claims

1. A cancer therapeutic agent for a cancer tissue containing CD209-positive cells, the cancer therapeutic agent containing a gel particle containing a modified polysaccharide having hydrophobic groups.

2. The cancer therapeutic agent according to claim 1, wherein the gel particle is a nanogel particle.

3. The cancer therapeutic agent according to claim 1 or 2, wherein a polysaccharide that forms the modified polysaccharide contains at least one member selected from the group consisting of pullulan, mannan, and dextran.

4. The cancer therapeutic agent according to any one of claims 1 to 3, wherein the polysaccharide that forms the modified polysaccharide contains pullulan.

5. The cancer therapeutic agent according to any one of claims 1 to 4, wherein the hydrophobic group contains a hydrophobic group having a sterol skeleton.

6. The cancer therapeutic agent according to any one of claims 1 to 5, wherein the modified polysaccharide has a weight average molecular weight of 5000 to 2,000,000.

7. The cancer therapeutic agent according to any one of claims 1 to 6, wherein the gel particle has a weight average particle size of 20 to 200 nm.

8. The cancer therapeutic agent according to any one of claims 1 to 7, wherein the gel particle contains drug(s).

9. The cancer therapeutic agent according to claim 8, wherein the drug contains at least one member selected from the group consisting of an adjuvant, a cancer antigen, an anticancer agent, and a nucleic acid medicine.

10. The cancer therapeutic agent according to any one of claims 1 to 9, wherein the CD209-positive cells are macrophages.

11. The cancer therapeutic agent according to any one of claims 1 to 10, for use in administration to a patient having cancer tissue(s) containing CD209-positive cells, the patient being selected from patients with cancer tissue(s).

12. The cancer therapeutic agent according to any one of claims 1 to 11, for use in combination with drug(s).

13. A delivery carrier to CD209-positive cells, containing a gel particle containing a modified polysaccharide having hydrophobic groups.

14. The delivery carrier according to claim 13, wherein the CD209-positive cells are present in cancer tissue.

15. An agent for detecting a tissue containing CD209-positive cells, containing the delivery carrier according to claim 13 or 14 and a contrast agent.

16. A companion diagnostic drug for the cancer therapeutic agent according to any one of claims 1 to 12, containing CD209-binding molecules.
